# EUROPEAN PATENT SPECIFICATION

(11) **EP 3 134 140 B1**
(45) Date of publication and mention of the grant of the patent: **30.01.2019**
(21) Application number: 15717713.0
(22) Date of filing: 27.03.2015
(51) Int. Cl.: A61L 31/02, A61L 31/14

(54) **BIOERODIBLE STENT**
BIOERODIERBARER STENT
ENDOPROTHÈSE BIOÉRODABLE

(30) Priority: 22.04.2014 US 201414258290
(43) Date of publication of application: 01.03.2017
(73) Proprietor: Medtronic Vascular Inc., Santa Rosa, CA 95403 (US)
(72) Inventor: PULUGURTHA, Syamala R., Santa Rosa, California 95403 (US)
(74) Representative: Zimmermann & Partner Patentanwälte mbB
(86) International application number: PCT/US2015/023092
(87) International publication number: WO 2015/164028

(56) References cited:
- WO-A2-2008/036548
- WO-A2-2009/079389
- US-A1- 2008 161 906

## Description

### FIELD OF THE INVENTION

The invention relates generally to temporary endoluminal prostheses for placement in a body lumen, and more particularly to stents that are bioerodible.

### BACKGROUND OF THE INVENTION

A wide range of medical treatments exist that utilize "endoluminal prostheses." As used herein, endoluminal prostheses is intended to cover medical devices that are adapted for temporary or permanent implantation within a body lumen, including both naturally occurring and artificially made lumens, such as without limitation: arteries, whether located within the coronary, mesentery, peripheral, or cerebral vasculature; veins; gastrointestinal tract; biliary tract; urethra; trachea; hepatic shunts; and fallopian tubes.

Accordingly, a wide assortment of endoluminal prostheses have been developed, each providing a uniquely beneficial structure to modify the mechanics of the targeted lumen wall. For example, stent prostheses are known for implantation within body lumens to provide artificial radial support to the wall tissue, which terms the various lumens within the body, and often more specifically, for implantation within the blood vessels of the body.

Essentially, stents that are presently utilized are made to be permanently or temporarily implanted. A permanent stent is designed to be maintained in a body lumen for an indeterminate amount of time and is typically designed to provide long term support for damaged or traumatized wall tissues of the lumen. There are numerous conventional applications for permanent stents including cardiovascular, urological, gastrointestinal, and gynecological applications. A temporary stent is designed to be maintained in a body lumen for a limited period of time in order to maintain the patency of the body lumen, for example, after trauma to a lumen caused by a surgical procedure or an injury.

Permanent stents, over time, may become encapsulated and covered with endothelium tissues, for example, in cardiovascular applications, causing irritation to the surrounding tissue. Further, if an additional interventional procedure is ever warranted, a previously permanently implanted stent may make it more difficult to perform the subsequent procedure.

Temporary stents, on the other hand, preferably do not become incorporated into the walls of the lumen by tissue ingrowth or encapsulation. Temporary stents may advantageously be eliminated from body lumens after an appropriate period of time, for example, after the traumatized tissues of the lumen have healed and a stent is no longer needed to maintain the patency of the lumen.

Bioerodible, bioabsorbable, bioresorbable, and biodegradable stents have been used as such temporary stents. For example, stents made of biodegradable polymers or magnesium have been proposed. However, some of these temporary stents may not provide sufficient strength to support the lumen when first implanted or may degrade too quickly or slowly. Accordingly, there is a need for a temporary stent with sufficient radial strength for initial support of the lumen and a controlled erosion after implantation.
US 2008/0161906 A1 describes bioerodible endoprostheses and methods of making the same. WO 2008/036548 A2 describes endoprostheses.

### BRIEF SUMMARY OF THE INVENTION

Disclosed herein are composite wires for a bioerodible helically wrapped wire stent according to claims 1 and 11.

Embodiments hereof also relate to a helically wrapped wire stent. The wire of the helically wrapped wire stent includes an inner member, an intermediate member surrounding the inner member, and an outer member surrounding the intermediate member. The inner member is made from a first metal that is less noble than a second metal of the intermediate member. The outer member is made from a third metal that is also less noble than the second metal of the intermediate member. In an embodiment, the inner member comprises magnesium or a magnesium alloy, the intermediate member comprises silver, and the outer member comprises molybdenum, tantalum, or tungsten. In an embodiment, a biodegradable polymer coating surrounds the outer member.

Embodiments hereof also relate to a bioerodible helically wrapped wire stent including an inner member having an outer surface, an intermediate member surrounding the inner member such that an inner surface of the intermediate member contacts the outer surface of the inner member, and an outer member deposited in recesses of the intermediate member. The inner member comprises a first biocompatible metal, the intermediate member comprises a second biocompatible metal, and the outer member comprises a third biocompatible member. The first biocompatible metal is less noble than the second biocompatible metal such that galvanic corrosion takes place between the inner member and the intermediate member and the second biocompatible metal is less noble than the third biocompatible metal such that galvanic corrosion takes place between the intermediate member and the outer member.

### BRIEF DESCRIPTION OF DRAWINGS

The foregoing and other features and advantages of the invention will be apparent from the following description of embodiments hereof as illustrated in the accompanying drawings. The accompanying drawings, which are incorporated herein and form a part of the specification, further serve to explain the principles of the invention and to enable a person skilled in the pertinent art to make and use the invention. The drawings are not to scale.
FIG. 1 is a schematic illustration of stent according to an embodiment hereof.
FIG. 2 is schematic cross-sectional view taken along line A-A of FIG. 1.
FIGS. 3-6 are schematic illustrations of steps in a method of forming the stent of FIG. 1.
FIG. 7 is a schematic illustration of a stent in accordance with another embodiment hereof.
FIG. 8 is a schematic cross-sectional view taken along line B-B of FIG. 7.
FIG. 8A is a schematic longitudinal cross-sectional view taken along line D-D of FIG. 7.
FIG. 9 is a schematic illustration of a composite wire used in a method of forming the stent of FIG. 7.
FIG. 10 is a schematic illustration of a stent in accordance with another embodiment hereof.
FIG. 11 is a schematic longitudinal cross-sectional view taken along line C-C of FIG. 10.
FIG. 11A is a schematic longitudinal cross-sectional view of another embodiment taken along line C-C of FIG. 10.
FIG. 12 is a schematic longitudinal cross-sectional view taken along line C-C of FIG. 10 with a coating added to the stent.
FIG 13 is a schematic longitudinal cross-section view of a portion of a composite wire used in a method of forming the stent of FIG. 10.
FIG. 14 is a schematic longitudinal cross-sectional view of the composite wire of FIG. 13 with recesses formed therein.

### DETAILED DESCRIPTION OF THE INVENTION

Specific embodiments of the present invention are now described with reference to the figures, wherein like reference numbers indicate identical or functionally similar elements.

As used herein "biocompatible" means any material that does not cause injury or death to the patient or induce an adverse reaction in the patient when placed in intimate contact with the patient's tissues. Adverse reactions include inflammation, infection, fibrotic tissue formation, cell death, or thrombosis.

The term "bioerodible" or "erodible" means a material or device, or portion thereof, that exhibits substantial mass or density reduction or chemical transformation after it is introduced into a patient, *e*.*g*., a human patient. Mass reduction can occur by, *e*.*g*., dissolution of the material that forms the device, fragmenting of the endoprosthesis, and/or galvanic reaction. Chemical transformation can include oxidation/reduction, hydrolysis, substitution, and/or addition reactions, or other chemical reactions of the material from which the device, or a portion thereof, is made. The erosion can be the result of a chemical and/or biological interaction of the device with the body environment, *e*.*g*., the body itself or body fluids, into which the device is implanted and/or erosion can be triggered by applying a triggering influence, such as a chemical reactant or energy to the device. The terms "bioresorbable" and "bioabsorbable" are often used as synonymous with "bioerodible" and may be used as such in the present application. Generally, this application will use the term "bioerodible" due to the nature of the erosion described in more detail below. However, the materials described may be described as bioabsorbable or bioresorbable as well.

As used herein, the term "biodegradable" means a material or device that will degrade over time by the action of enzymes, by hydrolytic action and/or by other similar mechanisms in the human body. Biodegradable is used broadly such that it may also refer to a material that is "bioerodible," however, the term biodegradable is generally broader such that it includes materials that are degradable but are not necessarily absorbed into the human body.

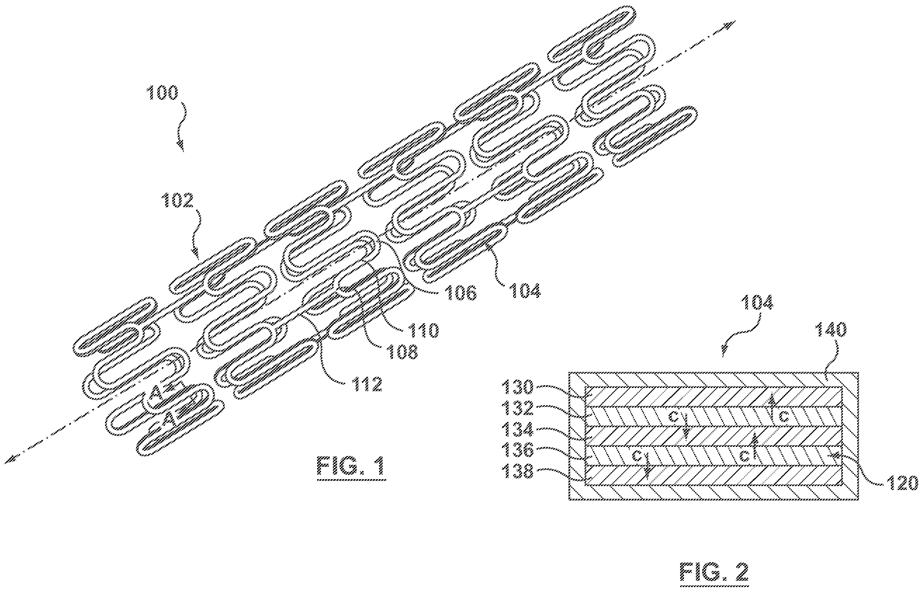

In an embodiment hereof shown in FIGS. 1 and 2, an endoluminal prosthesis or stent 100 is a patterned tubular device that includes a plurality of radially expandable cylindrical rings 102. Cylindrical rings 102 are formed from struts 104 formed in a generally sinusoidal pattern including peaks 106, valleys 108, and generally straight segments 110 connecting peaks 106 and valleys 108. Peaks 106 and valleys 108 may also be collectively referred to as bends or crowns. Connecting links 112 may be included to connect adjacent cylindrical rings 102 together. In FIG. 1, connecting links 112 are shown as generally straight links connecting a peak 106 of one ring 102 to a valley 108 of an adjacent ring 102. However, connecting links 112 may connect a peak 106 of one ring 102 to a peak 106 of an adjacent ring 112, or a valley 108 to a valley 108, or a straight segment 110 to a straight segment 110. Further, connecting links 112 may be curved. Connecting links 112 may also be excluded, with a peak 106 of one ring 102 being directly attached to a valley 108 of an adjacent ring 102, such as by welding, soldering, or the manner in which stent 100 is formed, such as by etching the pattern from a flat sheet or a tube.

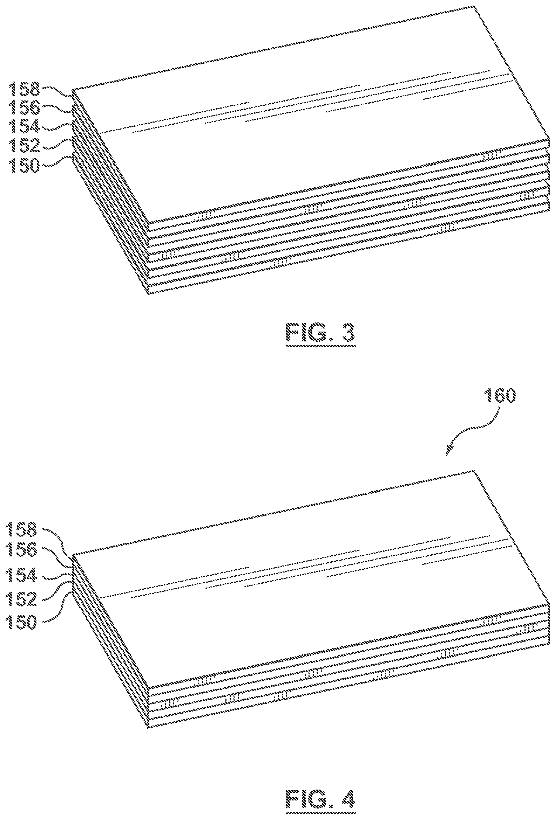

Stent 100 of FIG. 1 is merely an exemplary stent and stents of various forms and methods of fabrication can be used in accordance with various embodiments of the present invention. An example of a method of making stent 100 will be described with respect to FIGS. 3-6. However, other methods of making stent 100 may be used provided the resulting stent 100 include struts 104 as described in more detail below.

In accordance with various embodiments hereof, struts 104 of stent 100 include a laminate 120 comprising several layers of material. FIG. 2 shows a cross section of an embodiment of a strut 104 of stent 100. As shown in FIG. 2, laminate 120 includes a first metal layer 130, a second metal layer 132, a third metal layer 134, a fourth metal layer 136, and a fifth metal layer 138. The numbering of the layers is used for convenience and does not imply any particular orientation except as specified in further detail herein. The metal layers of laminate 120 are arranged such the laminate 120 corrodes in a certain pattern and timing when implanted into the body. In the embodiment of FIGS. 1-2, a coating 140 is disposed around laminate 120. In an embodiment, coating 140 may be a biodegradable polymeric material. Examples of biodegradable polymers for use in embodiments of the present invention, include, but are not limited to: poly(α-hydroxy acids), such as, polycapro lactone (PCL), poly(lactide-co-glycolide) (PLGA), polylactide (PLA), and polyglycolide (PGA), and combinations and blends thereof, PLGA-PEG (polyethylene glycol), PLA-PEG, PLA-PEG-PLA, polyanhydrides, trimethylene carbonates, polyorthoesters, polyaspirins, polyphosphagenes, and tyrozine polycarbonates. Coating 140 delays exposure of laminate 120 to tissues and fluids in the human body, thereby delaying the corrosion of laminate 120 described in detail below.

In the embodiment of FIGS. 1-6, first metal layer 130 and fifth metal layer 138 may also be referred to as "outer layers". Further, second metal layer 132 and fourth metal layer 126 may be referred to as "intermediate layers", with third metal layer 134 being referred to an "inner layer". The materials of the layers of laminate 120 are selected such that a galvanic coupling occurs between adjacent layers. A galvanic coupling occurs when there is a potential difference that occurs between two unlike metals in the presence of an electrolytic solution. In the present embodiment, galvanic coupling occurs because there is a potential difference between the materials of adjacent layers of laminate 120 in the presence of bodily fluids when the stent 100 is deployed in a body lumen. In a galvanic couple, the higher resistance or more noble metal turns cathodic, and may also be referred to as the cathode or less active material. The less resistant or less noble metal becomes anodic, and may also be referred to as the anode or active material. Typically, the cathodic material undergoes little or no corrosion in a galvanic couple, while the anodic material undergoes corrosion. Due to the unlike metals that are involved and the electric currents, the type of corrosion is referred to as two-metal or galvanic corrosion. The rate of corrosion is determined by the difference in electrolytic potential between the metals. The greater difference in the electrolytic potential between the metals, the more likelihood that corrosion will progress faster. The electrolytic difference can be measured by the difference in voltage potential between the materials, which may be measured against a Standard Hydrogen Electrode (SHE). The potential difference between an anode and a cathode can be measured by a voltage measuring device. The absolute potential of the anode and cathode cannot be measured directly. Defining a standard electrode, such as hydrogen, all other potential measurements can be made against this standard electrode. If the standard electrode potential is set to zero, the potential difference measured can be considered as the absolute potential. Accordingly, a metal's Standard Electrode Potential (SEP) is the potential difference measured between the metal and the Standard Hydrogen Electrode (SHE). Although the present application explains the electrolytic or potential difference with reference to a SHE, the SHE is a reference selected for convenience because most available literature includes lists on the subject of potential differences with respect to the SHE. Of course, lists also exist with potential differences compared to other standard electrodes, such as, for example, gold.

In an embodiment, third metal layer 134 (inner layer) is made of magnesium or a magnesium alloy. Magnesium in some literature is identified as having a Standard Electrode Potential of about -2.37 Volts. This value for magnesium depends on various measurement factors and conditions which could affect the value and is used herein only to show exemplary SEP differences between the materials described herein. Magnesium and magnesium alloys are also known to be bioabsorbable when used in a stent absent galvanic corrosion with adjacent metal layers. In other embodiments, materials such as iron or zinc may be used for the third metal layer 134.

In an embodiment, second metal layer 132 (intermediate layer) and fourth metal layer 136 (intermediate layer) are each made of silver. Silver in some literature is identified as having a Standard Electrode Potential of about 0.80 Volts. This value for silver depends on various measurement factors and conditions which could affect the value and is used herein only to show exemplary SEP differences between the materials described herein.

In an embodiment, first metal layer 130 (outer layer) and fifth metal layer 138 (outer layer) are each made of molybdenum. Molybdenum in some literature is identified as having a Standard Electrode Potential of -0.20 Volts. In other embodiments, materials such as tungsten (SEP ≈ -0.58) and tantalum (SEP ≈ -0.60) may be used for the first metal layer 132 and fifth metal layer 138. These SEP values depend on various measurement factors and conditions which could affect the values and are being used herein only to show exemplary SEP differences between materials described herein.

Thus, in the embodiment described above, the magnesium third layer 134 is less noble (more active) than the silver second and fourth layers 132, 136 which are in contact with magnesium third layer 134. Thus, the magnesium third layer 134 acts as an anode and experiences galvanic corrosion as a result of its contact with silver second and fourth layers 132, 136. Similarly, first and fifth layers 130, 138 are less noble (more active) and are in contact with second and fourth layers 132, 136, respectively. Thus, first and fifth layers 130, 138 act as an anode and experience galvanic corrosion as of result of their contact with silver second and fourth layers 132, 136, respectively. Accordingly, corrosion between the layers acts in the direction of arrows "C" shown in FIG. 2.

As described above, coating 140 is disposed around laminate 120. In an embodiment, coating 140 is a biocompatible, biodegradable polymer. After stent 100 is implanted within a body lumen, coating 140 prevents bodily fluid, such as blood in a blood vessel, from contacting laminate 120 until coating 140 at least partially degrades. Thus, the galvanic corrosion between the layers of laminate 120, as described above, is delayed until laminate 120 is exposed to the bodily fluid. Thus, coating 140 delays the galvanic corrosion. Accordingly, the material and thickness of coating 140 may be selected to customize when erosion of laminate 120 will begin.

Similarly, the materials and thicknesses of the layers of laminate 120 may be selected to customize the amount of time it takes for stent 100 to erode after implantation within the body lumen. In an embodiment, the third layer 134 is thicker than each of the second layer 132 and the fourth layer 136, the second layer 132 is thicker than the adjacent first layer 130, and the fourth layer 136 is thicker than the adjacent fifth layer 138. In an embodiment, first and fifth layers 130, 138 are in the range of 1.70 µm to 3.81 µ m (0.000067inch - 0.00015 inch) in thickness, second and fourth layers 132, 136 are in the range of 4.06 µm to 8.89 µ m (0.00016 inch - 0.00035 inch) in thickness, and third layer 134 is in the range of 102 µm to 114 µm (0.0040 inch - 0.0045 inch) in thickness. Further, coating 140 may be in the range of 1 µm - 2 µm in thickness. Although specific thicknesses are provided, different thicknesses may be used depending on where the stent 100 is implanted, the desired characteristics of stent 100, the desired length of delay before bodily fluids contact the laminate 120, the desired time for stent 100 to degrade/erode, and other factors
known to those skilled in the art. In an embodiment, stent 100 implanted in a coronary artery erodes/degrades completely in 30 to 90 days.

FIGS. 3-6 show an embodiment of a method of making stent 100. In the embodiment shown in FIGS. 3-6, five sheets or layers of material 150, 152, 154, 156, 158 are stacked, as shown in FIG. 3. In particular, first sheet 150 corresponds to first layer 130 of stent 100, second sheet 152 corresponds to second layer 132 of stent 100, third sheet 154 corresponds to third layer 134 of stent 100, fourth sheet 156 corresponds to fourth layer 136 of stent 100, and fifth sheet 158 corresponds to fifth layer 138 of stent 100. Thus, in an embodiment, first and fifth sheets 150, 158 may be molybdenum, tungsten, or tantalum, second and fourth sheets 152, 156 may be silver, and third sheet 154 may be magnesium, iron, or zinc, or alloys thereof.

The five sheets 150, 152, 154, 156, 158 are then pressed together to form a laminate 160, as shown in FIG. 4. The sheets may be pressed together by hot-isostatic pressing, cold rolling, or other methods to press swage or compression weld the sheets together. Other steps can be used to remove latent stresses from the sheets.

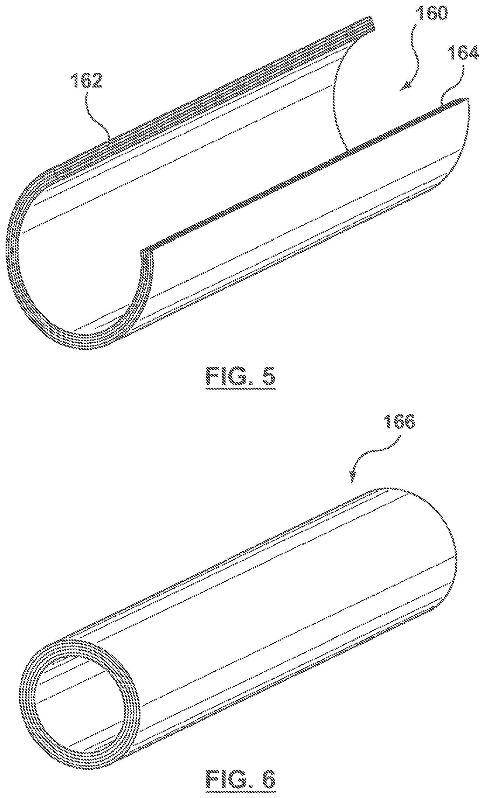

The laminate 160 may then be rolled such that a first longitudinal edge 162 and a second longitudinal edge 164 are rolled towards each other, as shown in FIG. 5. First longitudinal edge 162 and second longitudinal edge 164 may then be attached to each other, such as by welding, soldering, fusion, adhesive, or other various methods, thereby forming laminate tube 166, as shown in FIG. 6. Laminate tube 166 may then be processed such that portions of laminate tube 166 are removed and the remaining portions are in the form of stent 100 shown in FIG. 1. While the precise nature of this processing is not restricted, in one embodiment, the processing may be effected by a computer programmable laser cutting system which operates by: (i) receiving the laminate tube; (ii) moving the laminate tube longitudinally and rotationally under a laser beam to selectively remove portions of the laminate tube; and (iii) cutting stent sections of a desirable length for stent 100. A suitable laser cutting system known in the art is the LPLS-100 Series Stent Cutting Machine. Those skilled in the art would recognize that other methods of removing portions of the laminate tube may be used, such as, but not limited to, chemical etching and electron discharge machining can be used. Further, those skilled in the art would recognize that the stent pattern may be laser-cut or otherwise etched into the laminate 160 prior to laminate 160 being rolled into a tubular shape (*i*.*e*., while laminate 160 is flat). The resulting two-dimensional stent pattern may then be rolled into a tube, with opposing longitudinal edges being welded, fused, soldered, or otherwise bonded to each other to form stent 100.

With the pattern of stent 100 formed from laminate 160 and in a tubular form, stent 100 may be covered by coating 140. Stent 100 may be coated by coating 140 by dipping, spraying, painting, or other methods known to those skilled in the art.

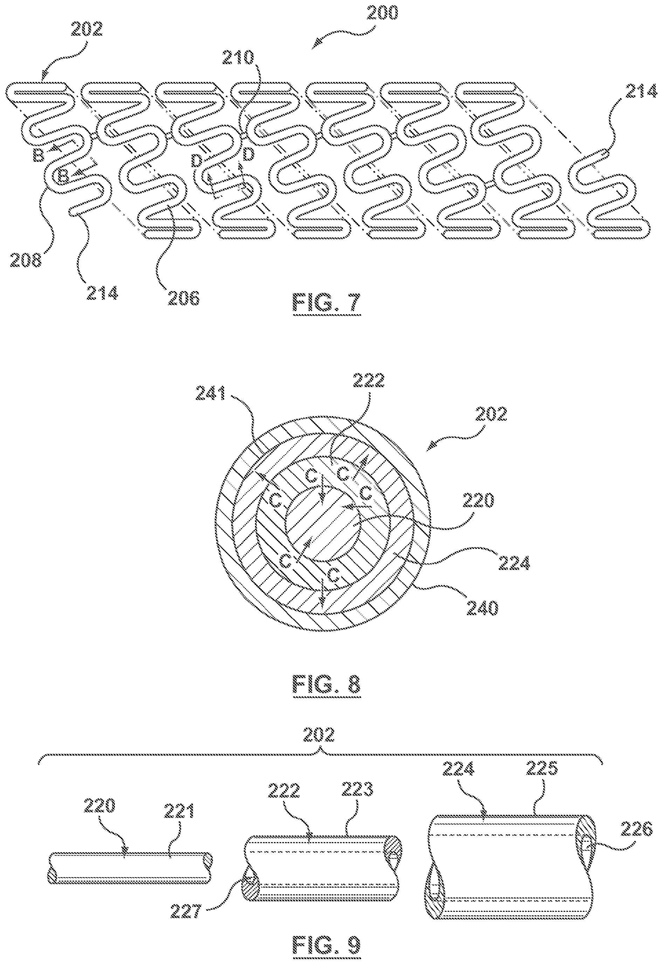

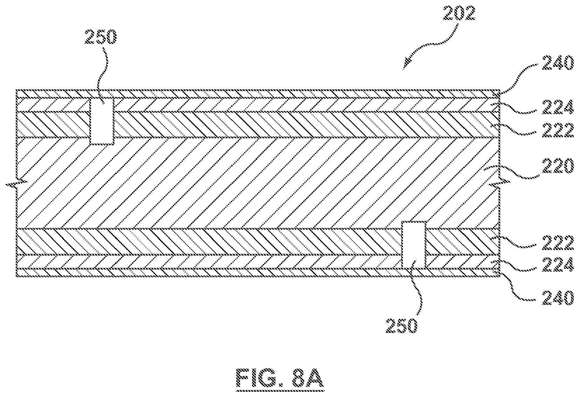

Another embodiment of a stent 200 disclosed herein is shown in FIGS. 7-9. In particular, stent 200 is formed from a wire 202, wherein the wire 202 is formed of an inner member 220, an intermediate member 222, and an outer member 224, as shown in FIG. 8. In an embodiment, wire 202 may also include a coating 240 disposed around outer member 224. The term "wire" as used herein means an elongated element or filament or group of elongated elements or filaments and is not limited to a particular cross-sectional shape or material, unless so specified. In the embodiment shown in FIG. 7, wire 202 is formed into a series of generally sinusoidal waveforms including generally straight segments or struts 206 joined by bent segments or crowns 208 and the waveform is helically wound to form a generally tubular stent 200. In the embodiment shown in FIG. 7, selected crowns 208 of longitudinally adjacent sinusoids may be joined by, for example, fusion points 210. Further, ends 214 of wire 202 may be welded, crimped or otherwise connected to other portions of wire 202 such that the ends 214 are not free ends. Ends 214 may alternatively be provided as free ends, as shown in FIG. 7. The invention hereof is not limited to the pattern shown in FIG. 7. Wire 202 of stent 200 can be formed into any pattern suitable for use as a stent. Further, instead of a single length of wire formed into a stent pattern, a plurality of wires may be formed into a two-dimensional waveform and wrapped into individual cylindrical elements. The cylindrical elements may then be aligned along a common longitudinal axis and joined to form the stent.

As shown in FIG. 8, wire 202 of stent 200 is a composite wire which includes inner member 220, intermediate member 222 surrounding inner member 220, and outer member 224 surrounding intermediate member 222. Accordingly, as shown in FIGS. 8-9, an inner surface 227 of intermediate member 222 surrounds and is in contact with an outer surface 221 of inner member 220. Similarly, an inner surface 226 of outer member 224 surrounds and is in contact with an outer surface 223 of intermediate member 222. If a coating 240 is used, an inner surface 241 of coating 240 surrounds and is in contact with an outer surface 225 of outer member 224. As described above with respect to the embodiment of FIGS. 1-6, materials for inner member 220, intermediate member 222, and outer member 224 are selected to customize erosion of wire 202 due to galvanic corrosion.

In an embodiment, inner member 220 is made of magnesium or a magnesium alloy. Magnesium is identified in some literature as having a Standard Electrode Potential of about - 2.37 Volts. Magnesium and magnesium alloys are also known to be bioabsorbable when used in a stent absent galvanic corrosion with adjacent metal layers. In other embodiments, materials such as zinc and iron may be used for inner member 220. In an embodiment, intermediate member 222 is made of silver. Silver has been identified in some literature as having a Standard Electrode Potential of about 0.80 Volts. In an embodiment, outer member 224 is made of molybdenum. Molybdenum is identified in some literature as having a Standard Electrode Potential of about -0.20 Volts. In other embodiments, materials such as tungsten (SEP ≈ -0.58) and tantalum (SEP ≈ -0.60) may be used for outer member 224. The SEP values listed above depend on various measurement factors and conditions which could affect the values and are being used herein only to show exemplary SEP differences between materials described herein.

Thus, in the embodiment described above, inner member 220 is less noble than intermediate member 222, with inner surface 227 of intermediate member 222 in contact with outer surface 221 of inner member 220. Thus, inner member 220 acts as an anode with respect to intermediate member 222 and experiences galvanic corrosion as a result of its contact with intermediate member 222. Similarly, outer member 224 is less noble and is in contact with intermediate member 222. Thus, outer member 224 acts as an anode with respect to intermediate member 222 and experiences galvanic corrosion as of result of its contact with intermediate layer 222. Accordingly, corrosion between the members acts in the direction of arrows "C" shown in FIG. 8.

As described above, coating 240 may be disposed around outer member 224 of wire 202. In an embodiment, coating 240 is a biocompatible, biodegradable polymer. Examples of biodegradable polymers for use in embodiments of the present invention, include, but are not limited to: poly(α-hydroxy acids), such as, polycapro lactone (PCL), poly(lactide-co-glycolide) (PLGA), polylactide (PLA), and polyglycolide (PGA), and combinations and blends thereof, PLGA-PEG (polyethylene glycol), PLA-PEG, PLA-PEG-PLA, polyanhydrides, trimethylene carbonates, polyorthoesters, polyaspirins, polyphosphagenes, and tyrozine polycarbonates. After stent 200 is implanted within a body lumen, coating 240 prevents bodily fluid, such as blood in a blood vessel, from contacting wire 202 until coating 240 at least partially degrades. As described above, bodily fluids act as the electrolytic solution required for galvanic corrosion between layers of dissimilar metals. Thus, the galvanic corrosion between the members of wire 202, as described above, is delayed until exposure to the bodily fluid. Thus, coating 240 delays the galvanic corrosion. Accordingly, the material and thickness of coating 240 may be selected to customize when erosion of wire 202 will begin.

Further, because the embodiment of FIGS. 7-9 is in the form of a wire, bodily fluids contact only outer member 224 until outer member 224 and intermediate member 222 degrade *in situ.* When outer member 224 at least partially degrades, bodily fluids reach intermediate member 222, thereby causing galvanic corrosion between outer member 224 and intermediate member 222. Similarly, when intermediate member 222 at least partially degrades, bodily fluids reach inner member 220, thereby causing galvanic corrosion between intermediate member 222 and inner member 220. In order to accelerate degradation of stent 200, if desired, notches or openings 250 may be provided through the outer member 224 and intermediate member 222, as shown in FIG. 8A. Openings 250 permit bodily fluids to reach intermediate member 222 after degradation of polymer coating 240 such that galvanic corrosion can begin between outer member 224 and intermediate member 222. Similarly, openings 250 permit bodily fluids to reach inner member 220 such that galvanic corrosion can begin between intermediate member 222 and inner member 220. The size, quantity, and location of openings 250 may be varied to customize the rate, location, and direction of corrosion. For example, and not by way of limitation, a stent with more openings 250 erodes quicker than a comparable stent with relatively less openings 250. Similarly, more openings 250 in an area of the stent can lead to a particular direction of the erosion. For example, and not by way of limitation, a stent with openings 250 towards the longitudinal ends of the stent and fewer or no openings toward the longitudinal center the stent would tend to erode from the longitudinal ends toward the center. Openings 250 may be laser drilled into wire 202 or formed by other methods. In an embodiment, openings 250 are approximately 20 microns in diameter. However, other sizes may be used.

Similarly, the materials and thicknesses of the members of wire 202 may be selected to customize the amount of time it takes for stent 200 to erode after implantation within the body lumen. In an embodiment, the inner member 220 is thicker than intermediate member 222, and intermediate member 222 is thicker than outer member 224. With reference to the embodiment of FIGS. 7-9, thickness means the wall thickness. In an embodiment, inner member 220 is in the range of 50.8 µm to 57.15 µm (0.0020 inch - 0.00225 inch) in thickness, intermediate member 222 is in the range of 0.00016 inch - 0.00035 inch in thickness, and outer member 224 is in the range of 0.000067 inch - 0.00015 inch in thickness. Further, coating 240 may be in the range of 1 µm - 2 µm in thickness. Although specific thicknesses are provided, different thicknesses may be used depending on where the stent 200 is implanted, the desired characteristics of stent 200, the desired length of delay before bodily fluids degrade coating 240, the desired time for stent 200 to degrade/erode, and other various factors. In an embodiment, stent 200 implanted in a coronary artery erodes/degrades completely in 30 to 90 days.

A method for forming stent 200 in accordance with an embodiment hereof includes utilizing a composite wire 202 having inner member 220, intermediate member 222, and outer member 224, as described above and shown schematically in FIG. 9. Composite wire 202 may be formed by any suitable method of forming composite wires. For example and not by way of limitation, composite wire 202 may be formed by a co-drawing process, extrusion, cladding, or any other suitable method..

Composite wire 202 is then shaped into a stent pattern. As discussed above, the stent pattern can be the pattern shown in FIG. 7 or any other suitable pattern formed from a wire. In an embodiment, shaping the composite wire 202 into the stent pattern shown in FIG. 7 generally includes the steps of forming composite wire 202 into a two dimensional waveform pattern followed by wrapping the pattern around a mandrel. The end result is a helical stent pattern formed onto a mandrel. Selected crowns 208 of the helical pattern may then be fused together and the stent may be removed from the mandrel. The step of shaping wire 202 into the stent pattern can be performed using various techniques. For example, and not by way of limitation, forming the wire 202 into a two dimensional waveform can be achieved using techniques described in U.S. Application Publication Nos. 2010/0269950 to Hoff et al., 2011/0070358 to Mauch et al., and 2013/0025339 to Costa et al.

Coating 240 may be applied to wire 202 by dipping, spraying, painting, or other various methods. Coating 240 may be applied after wire 202 is formed into the stent pattern or before wire 202 is formed into the stent pattern.

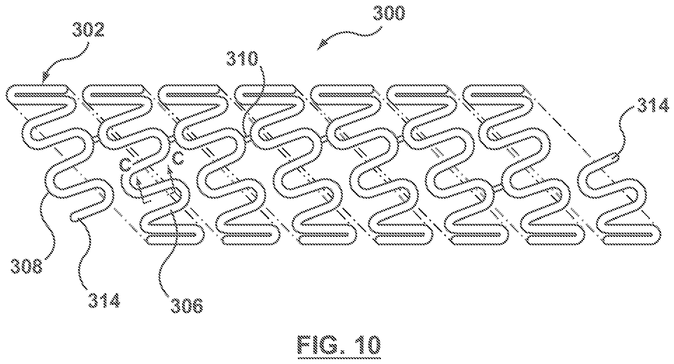

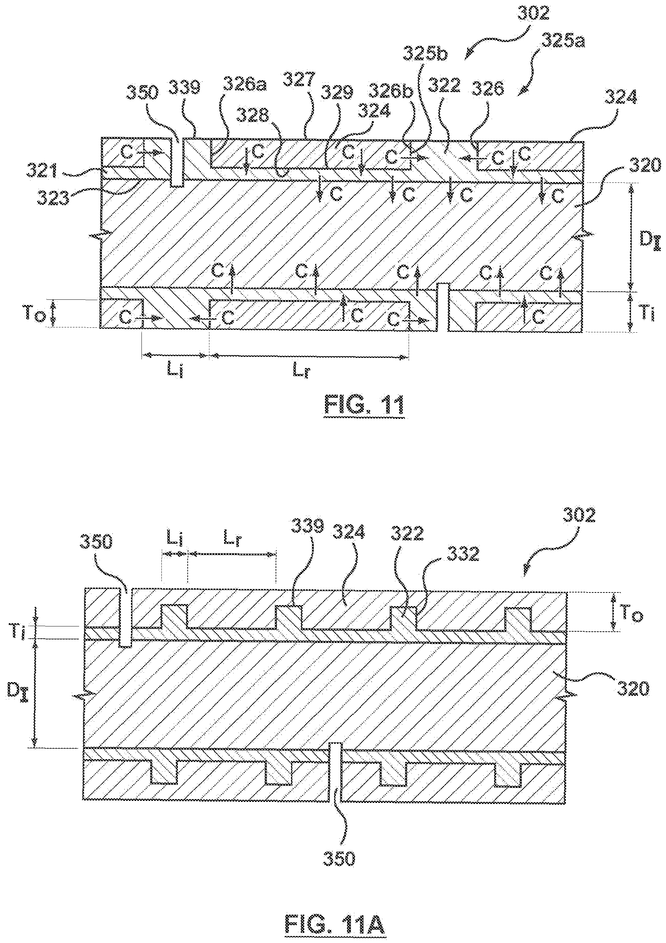

Another embodiment of a stent 300 disclosed herein is shown in FIGS. 10-14. In particular, stent 300 is formed from a wire 302. Wire 302 will be described in more detail with reference to FIGS. 11-12. The term "wire" as used herein means an elongated element or filament or group of elongated elements or filaments and is not limited to a particular cross-sectional shape or material, unless so specified. In the embodiment shown in FIG. 10, wire 302 is formed into a series of generally sinusoidal waveforms including generally straight segments or struts 306 joined by bent segments or crowns 308 and the waveform is helically wound to form a generally tubular stent 300. In the embodiment shown in FIG. 10, selected crowns 308 of longitudinally adjacent sinusoids may be joined by, for example, fusion points 310. Further, ends 314 of wire 302 may be welded, crimped or otherwise connected to other portions of wire 302 such that the ends 314 are not free ends. Ends 314 may alternatively be provided as free ends, as shown in FIG. 10. The invention hereof is not limited to the pattern shown in FIG. 10. Wire 302 of stent 300 can be formed into any pattern suitable for use as a stent. Further, instead of a single length of wire formed into a stent pattern, a plurality of wires may be formed into a two-dimensional waveform and wrapped into individual cylindrical elements. The cylindrical elements may then be aligned along a common longitudinal axis and joined to form the stent.

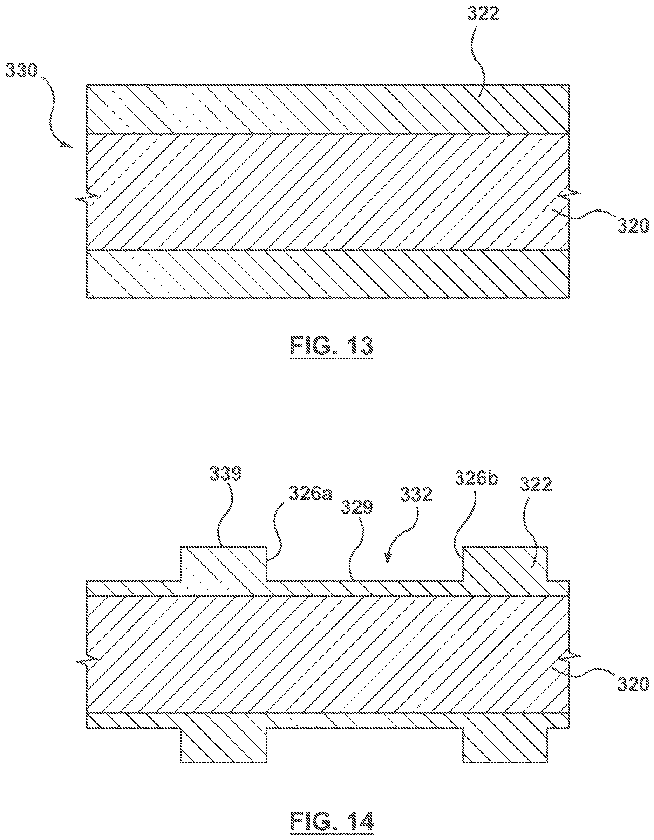

FIG. 11 shows a longitudinal cross-section of wire 302 of stent 300. Wire 302 includes an inner member 320, an intermediate member 322 including indentations, notches, or recesses 332 (shown in FIG. 14) and an outer member 324 disposed in recesses 332. In particular, intermediate member 322 surrounds inner member 320 such that an inner surface 323 of intermediate member 322 is in contact with an outer surface 321 of inner member 320. Recesses 332 in intermediate member 322 are defined by a first sidewall surface 326*a* and a second sidewall surface 326*b* of intermediate member 322. A bottom surface 329 of recess 332 extends between first sidewall surface 326*a* and second sidewall surface 326*b*. Bottom surface 329 is recessed from an outer surface 339 of intermediate member 322 where intermediate member 322 is not recessed. Although recesses 332 are shown with vertical sidewall surfaces 326 and a rectangular cross-section, that recesses 332 may be of any desired shape and sidewall surfaces 326*a*, 326*b* may be, for example, angled. Outer member 324 is disposed in recesses 332 of intermediate member 322. An inner surface 328 of outer member 324 is in contact with bottom surface 329 of recess 332. A first sidewall surface 325*a* of outer member 324 is in contact with first sidewall surface 326*a* of recess 332. A second sidewall surface 325*b* of outer member 324 is in contact with second sidewall surface 326*b* of recess 332. FIG. 11A shows a variation of FIG. 11 with outer member 324 deposited in recesses 332 and also covering outer surface 339 of intermediate member 322.

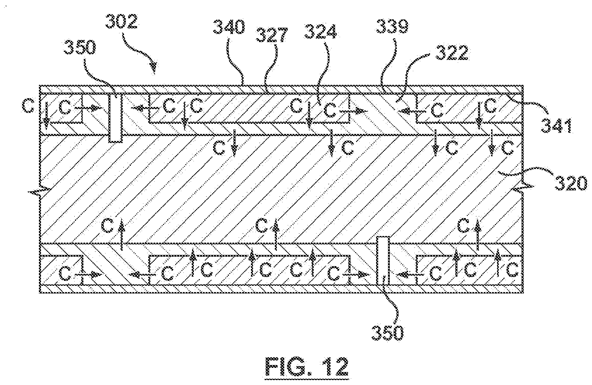

In an embodiment shown in FIG. 12, a coating 340 is used. In such an embodiment, an inner surface 341 of coating 340 surrounds and is in contact with an outer surface 327 of outer member 324 where outer member fills recesses 332 and is contact with outer surface 339 of intermediate member 322 at areas without a recess 332. Materials for inner member 320, intermediate member 322, and outer member 324 are selected to customize erosion of wire 302 due to galvanic corrosion.

In an embodiment, inner member 320 is made of magnesium or a magnesium alloy. Magnesium is identified in some literature as having a Standard Electrode Potential of about - 2.37 Volts. Magnesium and magnesium alloys are also known to be bioabsorbable when used in a stent absent galvanic corrosion with adjacent metal layers. In other embodiments, materials such as zinc and iron may be used for inner member 320. In an embodiment, intermediate member 322 is made of molybdenum. Molybdenum is identified in some literature as having a Standard Electrode Potential of about -0.20 Volts. In other embodiments, materials such as tungsten (SEP ≈ -0.58) and tantalum (SEP ≈ -0.60) may be used for intermediate member 322. In an embodiment, outer member 324 is made of silver. Silver is identified in some literature as having a Standard Electrode Potential of about 0.80 Volts. The SEP values listed above depend on various measurement factors and conditions which could affect the values and are being used herein only to show exemplary SEP differences between materials described herein.

Thus, in the embodiment described above, inner member 320 is less noble than intermediate member 322, with inner surface 323 of intermediate member 322 in contact with outer surface 321 of inner member 320. Thus, inner member 320 acts as an anode with respect to intermediate member and experiences galvanic corrosion as a result of its contact with the more noble intermediate member 322. Similarly, intermediate member 322 is less noble and is in contact with outer member 324 where outer surface 329 of intermediate member 322 contacts inner surface 328 of outer member 324 and where first and second side surfaces 326*a*, 326*b* of recesses 332 contact first and second side surfaces 325*a*, 325*b* of outer member 324. Thus, intermediate member 322 acts as an anode with respect to outer member 324 and experiences galvanic corrosion as of result of its contact with the more noble outer member 324. Accordingly, corrosion between the members acts in the direction of arrows "C" shown in FIGS. 11 and 12.

As described above, coating 340 may be disposed around intermediate member 322 and outer member 324 of wire 302, as shown in FIG. 12. In an embodiment, coating 340 is a biocompatible, biodegradable polymer. Examples of biodegradable polymers for use in embodiments of the present invention, include, but are not limited to: poly(α-hydroxy acids), such as, polycapro lactone (PCL), poly(lactide-co-glycolide) (PLGA), polylactide (PLA), and polyglycolide (PGA), and combinations and blends thereof, PLGA-PEG (polyethylene glycol), PLA-PEG, PLA-PEG-PLA, polyanhydrides, trimethylene carbonates, polyorthoesters, polyaspirins, polyphosphagenes, and tyrozine polycarbonates. After stent 300 is implanted within a body lumen, coating 340 prevents bodily fluid, such as blood in a blood vessel, from contacting wire 302 until coating 340 at least partially degrades. As described above, bodily fluids act as the electrolytic solution required for galvanic corrosion between layers of dissimilar metals. Thus, the galvanic corrosion between the members of wire 302, as described above, is delayed until exposure to the bodily fluid. Thus, coating 340 delays the galvanic corrosion. Accordingly, the material and thickness of coating 340 may be selected to customize when erosion of wire 302 will begin.

Further, because the embodiment of FIGS. 10-12 is in the form of a wire, bodily fluids initially only contact outer member 324 and intermediate member 322 until outer member 324 degrades *in situ.* Thus, bodily fluids do not contact the interface of intermediate member 322 and inner member 320 until intermediate member 322 at least partially degrades. Thus, galvanic corrosion between inner member 320 and intermediate member 322 does not occur until intermediate member at least partially degrades. In order to accelerate degradation of stent 300, if desired, notches or openings 350 may be provided through intermediate member 322 to inner member 320, as shown in FIGS. 11 and 12. Openings (not shown) may also be provided through outer member 324 and intermediate member 322 at the location of recesses 332, either in addition to openings 350 or in lieu thereof. Openings 350 permit bodily fluids to reach inner member 320 after degradation of polymer coating 340 such that galvanic corrosion can begin between intermediate member 322 and inner member 320. The size, quantity, and location of openings 350 may be varied to customize the rate, location, and direction of corrosion, as described above with respect to stent 200. Openings 350 may be laser drilled into wire 302 or formed by other methods. In an embodiment, openings 350 are approximately 20 microns in diameter. However, other sizes may be used.

Similarly, the materials and thicknesses of the members of wire 302 may be selected to customize the amount of time it takes for stent 300 to erode after implantation within the body lumen. In an embodiment of FIG. 11 or FIG. 12, the diameter D_{I} of inner member 320 is in the range of 101.6 µm to 114.3 µm (0.0040 inch - 0.0045 inch) in thickness. Further, the wall thickness Tᵢ of intermediate member 322 is in the range of 5.715 µm t o12.7 µm (0.000225 inch - 0.0005 inch), and the wall thickness Tₒ of outer member 324 is in the range of 4.064 µm to 8.89 µm (0.00016 inch - 0.00035 inch). Further, the length Lᵢ intermediate member 322 between recesses 332 may be approximately 127 µm (0.005 inch) and each recess 332 may have a length Lᵣ of approximately 254 µm (0.01 inch). Further, coating 340 of FIG. 12 may be in the range of 1 µm - 2 µm in thickness. In the variation shown in FIG. 11A, the diameter D_{I} of inner member 320 is in the range of 1.106 µm to 114.3 µm (0.0040 inch - 0.0045 inch) in thickness. Further, the wall thickness Tᵢ of intermediate member 322 from the outer surface of inner member to the bottom surface of recess is in the range of 1.143 µm to 8.128 µm (0.000045 inch - 0.00032 inch), and the wall thickness Tₒ of outer member 324 is in the range of 5.715 µm to 12.7 µm (0.000225 inch - 0.0005 inch). Further, the length Lᵢ intermediate member 322 between recesses 332 may be approximately 2.54 µm (0.0001 inch) and each recess 332 may have a length Lᵣ of approximately 254 µm (0.01 inch). Although specific sizes are provided, they are just examples and different sizes may be used depending on where the stent 300 is implanted, the desired characteristics of stent 300, the desired length of delay before bodily fluids degrade coating 340, the desired time for stent 300 to degrade/erode, and various other factors. In an embodiment, stent 300 implanted in a coronary artery erodes/degrades completely in 30 to 90 days.

A method for forming stent 300 in accordance with an embodiment hereof includes utilizing a composite wire 330 having inner member 320 and intermediate member 322, as shown in FIG. 13. Composite wire 330 may be formed, for example and not by way of limitation, by a co-drawing process, extrusion, cladding, or any other suitable method.

Recesses 332 are then formed in intermediate member 322 of composite wire 330, as shown in FIG. 14. Recesses 332 may be formed by various methods, such as, but not limited to, photolithography techniques or wet or dry etching. Outer layer 324 may then be deposited in recesses 332, resulting in wire 302 shown in FIG. 11. If desired, coating 340 may be applied to wire 302 by dipping, spraying, painting, or other various methods,
resulting in the wire shown in FIG. 12. Coating 340 may be applied after wire 302 is formed into the stent pattern or before wire 302 is formed into the stent pattern, as described in more detail below.

Wire 302 is then shaped into a stent pattern. As discussed above, the stent pattern can be the pattern shown in FIG. 10 or any other suitable pattern formed from a wire. In an embodiment, shaping the wire 302 into the stent pattern shown in FIG. 10 generally includes the steps of forming wire 302 into a two dimensional waveform pattern followed by wrapping the pattern around a mandrel. The end result is a helical stent pattern formed onto a mandrel. Selected crowns 308 of the helical pattern may then be fused together and the stent may be removed from the mandrel. The step of shaping wire 302 into the stent pattern can be performed using various techniques. For example, and not by way of limitation, forming the wire 302 into a two dimensional waveform can be achieved using techniques described in U.S. Application Publication Nos. 2010/0269950 to Hoff et al., 2011/0070358 to Mauch et al., and 2013/0025339 to Costa et al.

As noted above, the steps described above need not be performed in the particular order noted. For example, and not by way of limitation, the coating step may be performed after the wire 302 has been formed in to the stent pattern. Further, the steps of forming the recesses and filing the recessed with the material of the outer member may be performed after shaping the wire into the stent pattern, although it is preferable to perform these steps on the wire prior to shaping.

## Claims

1. A composite wire for a bioerodible helically wrapped wire stent comprising:
an inner member having an outer surface, the inner member comprising a first biocompatible metal;
an intermediate member surrounding the inner member such that an inner surface of the intermediate member contacts the outer surface of the inner member, the intermediate member comprising a second biocompatible metal; and
an outer member surrounding the intermediate member such that an inner surface of the outer member contacts an outer surface of the intermediate member, wherein the outer member comprises a third biocompatible metal,
wherein the first biocompatible metal is less noble than the second biocompatible metal such that galvanic corrosion takes place between the inner member and the intermediate member when exposed to bodily fluids and the third biocompatible metal is less noble than the second biocompatible metal such that galvanic corrosion takes place between the outer member and the intermediate member when exposed to bodily fluids.

2. The composite wire of claim 1, wherein the first biocompatible metal comprises magnesium, zinc, or iron, or alloys thereof.

3. The composite wire of claim 2, wherein the second biocompatible metal comprises silver.

4. The composite wire of claim 3, wherein the third biocompatible metal comprises molebdynum, tungsten, or tantalum.

5. The composite wire of claim 4, further comprising a biodegradable polymeric material surrounding the outer member.

6. The composite wire of claim 1, further comprising a biodegradable polymeric material surrounding the outer member and the intermediate member.

7. The composite wire of claim 1, wherein the second biocompatible metal comprises silver.

8. The composite wire of claim 1, wherein the third biocompatible metal comprises molebdynum, tungsten, or tantalum.

9. A bioerodible stent including the composite wire of any preceding claim, wherein the stent is shaped into a suitable pattern formed from the wire.

10. The bioerodible stent of claim 9, wherein the stent is a helically wrapped wire stent.

11. A composite wire for a bioerodible helically wrapped wire stent comprising:
an inner member having an outer surface, the inner member comprising a first biocompatible metal;
an intermediate member surrounding the inner member such that an inner surface of the intermediate member contacts the outer surface of the inner member, the intermediate member comprising a second biocompatible metal, wherein the intermediate member includes recesses formed therein; and
an outer member deposited in the recesses of the intermediate member, wherein the outer member comprises a third biocompatible metal,
wherein the first biocompatible metal is less noble than the second biocompatible metal such that galvanic corrosion takes place between the inner member and the intermediate member and the second biocompatible metal is less noble than the third biocompatible metal such that galvanic corrosion takes place between the intermediate member and the outer member.

12. The composite wire of claim 11, wherein the first biocompatible metal comprises magnesium, zinc, or iron, or alloys thereof; wherein optionally the second biocompatible metal comprises molebdynum, tungsten, or tantalum; wherein optionally the third biocompatible metal comprises silver; the bioerodible stent optionally comprising a biodegradable polymeric material surrounding the outer member and the intermediate member.

13. The composite wire of claim 11, further comprising a biodegradable polymeric material surrounding the outer member and the intermediate member.

14. The composite wire of claim 11, wherein the second biocompatible metal comprises molebdynum, tungsten, or tantalum.

15. The composite wire of claim 11, wherein the third biocompatible metal comprises silver.

## Patentansprüche

1. Verbunddraht für einen biologisch erodierbaren, spiralförmig gewickelten Drahtstent, umfassend:
ein Innenelement mit einer Außenoberfläche, wobei das Innenelement ein erstes biokompatibles Metall umfasst;
ein Zwischenelement, das das Innenelement so umgibt, dass eine Innenoberfläche des Zwischenelements die Außenoberfläche des Innenelements berührt, wobei das Zwischenelement ein zweites biokompatibles Metall umfasst; und
ein Außenelement, das das Zwischenelement so umgibt, dass eine Innenoberfläche des Außenelements eine Außenoberfläche des Zwischenelements berührt, wobei das Außenelement ein drittes biokompatibles Metall umfasst,
wobei das erste biokompatible Metall weniger Edelmetall als das zweite biokompatible Metall aufweist, so dass galvanische Korrosion zwischen dem Innenelement und dem Zwischenelement stattfindet, wenn sie Körperflüssigkeiten ausgesetzt sind, und das dritte biokompatible Metall weniger Edelmetall als das zweite biokompatible Metall aufweist, so dass galvanische Korrosion zwischen dem Außenelement und dem Zwischenelement stattfindet, wenn sie Körperflüssigkeiten ausgesetzt sind.

2. Verbunddraht nach Anspruch 1, wobei das erste biokompatible Metall Magnesium, Zink oder Eisen oder Legierungen davon umfasst.

3. Verbunddraht nach Anspruch 2, wobei das zweite biokompatible Metall Silber umfasst.

4. Verbunddraht nach Anspruch 3, wobei das dritte biokompatible Metall Molybdän, Wolfram oder Tantal umfasst.

5. Verbunddraht nach Anspruch 4, der ferner ein biologisch abbaubares Polymermaterial umfasst, das das Außenelement umgibt.

6. Verbunddraht nach Anspruch 1, der ferner ein biologisch abbaubares Polymermaterial umfasst, das das Außenelement und das Zwischenelement umgibt.

7. Verbunddraht nach Anspruch 1, wobei das zweite biokompatible Metall Silber umfasst.

8. Verbunddraht nach Anspruch 1, wobei das dritte biokompatible Metall Molybdän, Wolfram oder Tantal umfasst.

9. Biologisch erodierbarer Stent, der den Verbunddraht nach einem der vorstehenden Ansprüche einschließt, wobei der Stent zu einem geeigneten Muster, das aus dem Draht gebildet ist, geformt ist.

10. Biologisch erodierbarer Stent nach Anspruch 9, wobei der Stent ein spiralförmig gewickelter Drahtstent ist.

11. Verbunddraht für einen biologisch erodierbaren, spiralförmig gewickelten Drahtstent, umfassend:
ein Innenelement mit einer Außenoberfläche, wobei das Innenelement ein erstes biokompatibles Metall umfasst;
ein Zwischenelement, das das Innenelement so umgibt, dass eine Innenoberfläche des Zwischenelements die Außenoberfläche des Innenelements berührt, wobei das Zwischenelement ein zweites biokompatibles Metall umfasst, wobei das Zwischenelement darin ausgebildete Vertiefungen einschließt; und
ein Außenelement, das in den Vertiefungen des Zwischenelements angelagert ist, wobei das Außenelement ein drittes biokompatibles Metall umfasst,
wobei das erste biokompatible Metall weniger Edelmetall als das zweite biokompatible Metall aufweist, so dass galvanische Korrosion zwischen dem Innenelement und dem Zwischenelement stattfindet, und das zweite biokompatible Metall weniger Edelmetall aufweist als das dritte biokompatible Metall, so dass galvanische Korrosion zwischen dem Zwischenelement und dem Außenelement stattfindet.

12. Verbunddraht nach Anspruch 11, wobei das erste biokompatible Metall Magnesium, Zink oder Eisen oder Legierungen davon umfasst; wobei wahlweise das zweite biokompatible Metall Molybdän, Wolfram oder Tantal umfasst; wobei wahlweise das dritte biokompatible Metall Silber umfasst; wobei der biologisch erodierbare Stent wahlweise ein biologisch abbaubares Polymermaterial umfasst, das das Außenelement und das Zwischenelement umgibt.

13. Verbunddraht nach Anspruch 11, der ferner ein biologisch abbaubares Polymermaterial umfasst, das das Außenelement und das Zwischenelement umgibt.

14. Verbunddraht nach Anspruch 11, wobei das zweite biokompatible Metall Molybdän, Wolfram oder Tantal umfasst.

15. Verbunddraht nach Anspruch 11, wobei das dritte biokompatible Metall Silber umfasst.

## Revendications

1. Fil composite pour un stent à fil enveloppé de manière hélicoïdale bioérodable, comprenant :
un élément interne ayant une surface externe, l'élément interne comprenant un premier métal biocompatible ;
un élément intermédiaire entourant l'élément interne de telle sorte qu'une surface interne de l'élément intermédiaire vient en contact avec la surface externe de l'élément interne, l'élément intermédiaire comprenant un deuxième métal biocompatible ; et
un élément externe entourant l'élément intermédiaire de telle sorte qu'une surface interne de l'élément externe vient en contact avec une surface externe de l'élément intermédiaire, dans lequel l'élément externe comprend un troisième métal biocompatible,
dans lequel le premier métal biocompatible est moins noble que le deuxième métal biocompatible de telle sorte qu'une corrosion galvanique se produit entre l'élément interne et l'élément intermédiaire lors d'une exposition à des fluides corporels et le troisième métal biocompatible est moins noble que le deuxième métal biocompatible de telle sorte qu'une corrosion galvanique se produit entre l'élément externe et l'élément intermédiaire lors d'une exposition à des fluides corporels.

2. Fil composite selon la revendication 1, dans lequel le premier métal biocompatible comprend du magnésium, du zinc ou du fer, ou des alliages de ceux-ci.

3. Fil composite selon la revendication 2, dans lequel le deuxième métal biocompatible comprend de l'argent.

4. Fil composite selon la revendication 3, dans lequel le troisième métal biocompatible comprend du molybdène, du tungstène ou du tantale.

5. Fil composite selon la revendication 4, comprenant en outre un matériau polymère biodégradable entourant l'élément externe.

6. Fil composite selon la revendication 1, comprenant en outre un matériau polymère biodégradable entourant l'élément externe et l'élément intermédiaire.

7. Fil composite selon la revendication 1, dans lequel le deuxième métal biocompatible comprend de l'argent.

8. Fil composite selon la revendication 1, dans lequel le troisième métal biocompatible comprend du molybdène, du tungstène ou du tantale.

9. Stent bioérodable incluant le fil composite selon une quelconque revendication précédente, dans lequel le stent est mis en forme en un motif approprié formé à partir du fil.

10. Stent bioérodable selon la revendication 9, dans lequel le stent est un stent à fil enveloppé de manière hélicoïdale.

11. Fil composite pour un stent à fil enveloppé de manière hélicoïdale bioérodable, comprenant :
un élément interne ayant une surface externe, l'élément interne comprenant un premier métal biocompatible ;
un élément intermédiaire entourant l'élément interne de telle sorte qu'une surface interne de l'élément intermédiaire vient en contact avec la surface externe de l'élément interne, l'élément intermédiaire comprenant un deuxième métal biocompatible, dans lequel l'élément intermédiaire inclut des évidements formés en son sein ; et
un élément externe déposé dans les évidements de l'élément intermédiaire, dans lequel l'élément externe comprend un troisième métal biocompatible,
dans lequel le premier métal biocompatible est moins noble que le deuxième métal biocompatible de telle sorte qu'une corrosion galvanique se produit entre l'élément interne et l'élément intermédiaire et le deuxième métal biocompatible est moins noble que le troisième métal biocompatible de telle sorte qu'une corrosion galvanique se produit entre l'élément intermédiaire et l'élément externe.

12. Fil composite selon la revendication 11, dans lequel le premier métal biocompatible comprend du magnésium, du zinc ou du fer, ou des alliages de ceux-ci ; dans lequel éventuellement le deuxième métal biocompatible comprend du molybdène, du tungstène ou du tantale ; dans lequel éventuellement le troisième métal biocompatible comprend de l'argent ; le stent bioérodable comprenant éventuellement un matériau polymère biodégradable entourant l'élément externe et l'élément intermédiaire.

13. Fil composite selon la revendication 11, comprenant en outre un matériau polymère biodégradable entourant l'élément externe et l'élément intermédiaire.

14. Fil composite selon la revendication 11, dans lequel le deuxième métal biocompatible comprend du molybdène, du tungstène ou du tantale.

15. Fil composite selon la revendication 11, dans lequel le troisième métal biocompatible comprend de l'argent.
